# FASCICULE DE BREVET EUROPEEN

(11) **EP 1 449 501 B1**
(45) Date de publication et mention de la délivrance du brevet: **15.08.2007**
(21) Numéro de dépôt: 03075495.6
(22) Date de dépôt: 20.02.2003
(51) Int. Cl.: A61F 2/46

(54) **Dispositif de test pour tête de prothèse fémorale**
Testvorrichtung für Hüftgelenk-Endoprothese
Testing device for socket insert for a hip

(43) Date de publication de la demande: 25.08.2004
(73) Titulaire: Saphirwerk Industrieprodukte AG, 2555 Brügg (CH)
(72) Inventeur: Bäbler, Jean, 1700 Fribourg (CH); Burri, Jürg, 2572 Sutz (CH); Felber, Hans, 6052 Hergiswil (CH); Buntschu, Urs, 2562 Port (CH); Salvisberg, Werner, 3250 Lyss (CH)
(74) Mandataire: DTS Zürich

(56) Documents cités:
- WO-A-00/16066
- DE-A- 4 411 508
- US-B1- 6 176 140

## Description

La présente invention concerne un dispositif pour tester des têtes de prothèses fémorales, en particulier pour tester des têtes de prothèses réalisées en matériau céramique.

De manière connue, les prothèses fémorales comprennent une tige fémorale, destinée à être implantée dans le canal médullaire. La tige se termine par un embout à extrémité tronconique et est coiffée par emmanchement d'une tête fémorale ayant la forme générale d'une sphère. La boule est pourvue d'un perçage borgne et présente une forme complémentaire à celle de l'embout de la tige fémorale. Ces boules, qui sont le plus souvent réalisées en céramique, par exemple en ZrO₂, Al₂O₃, Si₃N₄, etc., sont soumises au cours de leur utilisation à des contraintes mécaniques élevées et doivent par conséquent répondre à des critères de fiabilités élevés.

En dépit du grand soin apporté à la fabrication de telles têtes, celles-ci comportent inévitablement des défauts microscopiques. Ces têtes doivent par conséquent être testées en fin de fabrication afin d'éliminer celles présentant des défauts qui dépassent une taille critique. Pour ce faire, plusieurs tests ont été envisagés.

Un premier test, dénommé « Proof test » selon la terminologie anglaise, consiste à appliquer une force régulière sur la surface intérieure du perçage tronconique de la boule, la force appliquée étant nettement inférieure à la force maximale provoquant la rupture de la boule. A titre d'exemple, ce test imposé par la "Food and Drug Administration" américaine conseille que les têtes fémorales devant être implantées satisfassent ce test et notamment résistent à une force minimum de 20kN.

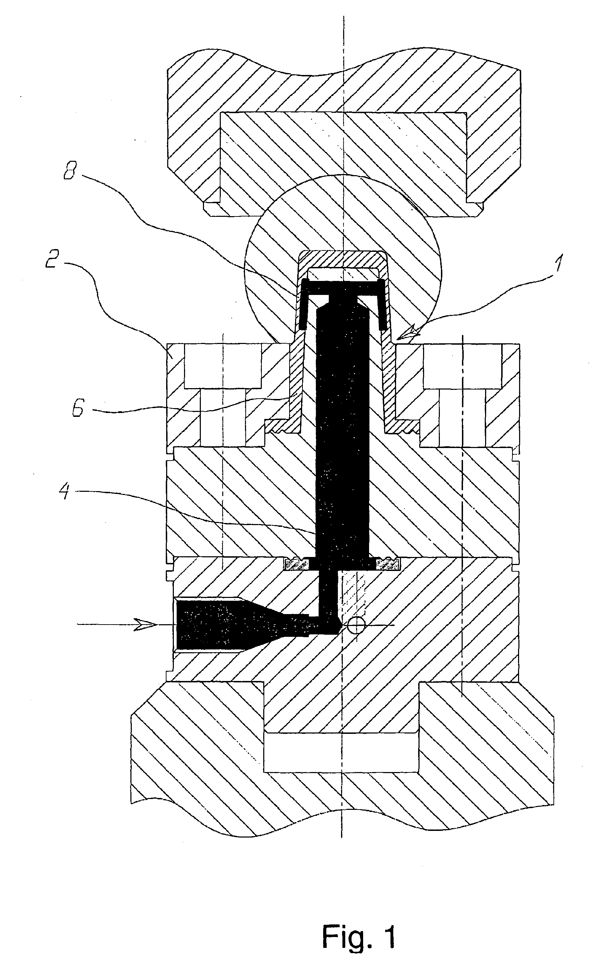

Ce test est généralement mis en oeuvre sur un appareil du type illustré schématiquement à la figure 1. Cet appareil comprend un fût tronconique 1 faisant saillie à partir d'une base 2. Le fût 1 comprend des canaux 4 de circulation d'un fluide sous pression, débouchant sur sa surface tronconique. Le fût est coiffé d'un manchon 6 en matière plastique. Le manchon 6 comprend dans sa partie intérieure une gorge annulaire 8 qui définit avec la surface tronconique du fût une chambre de pression dans laquelle circule le fluide sous pression. A l'endroit de la gorge 8, la paroi du manchon 8 est amincie et forme une membrane déformable qui peut s'appuyer contre la paroi intérieure du perçage. Le test des têtes fémorales est réalisé de la façon suivante. La tête est emmanchée sur le fût et est maintenue dans cette position par un contre-appui. Un fluide sous pression est alors introduit dans les canaux de circulation ménagés dans le fût pour produire la pression désirée sur la paroi latérale du perçage. La pression est transmise par l'intermédiaire de la membrane déformable du manchon. A titre indicatif, les pressions nécessaires pour la mise en oeuvre du « Proof test » sur ce genre d'appareil, sont de l'ordre de 650 bars.

Un deuxième test mis en oeuvre par les fabricants de têtes fémorales consiste à prélever des échantillons dans des lots de fabrication et à les soumettre à un test destructif visant à déterminer la résistance à la rupture moyenne par lot, par exemple en fonction de la forme du logement tronconique, de la qualité de la céramique, de la rugosité de surface et des micro-fissures provoquées par le procédé de fabrication.

L'appareil de test décrit ci-dessus ne permet pas d'utiliser des pressions suffisamment élevées pour déterminer la pression maximum à laquelle la boule se rompt, car le manchon en matière plastique ne résiste pas à ces pressions, qui peuvent dépasser 5000 bars. Il est donc nécessaire de recourir à un autre appareil pour réaliser ces tests. L'appareil utilisé pour cela comprend un fût muni d'une extrémité tronconique sur laquelle est emmanchée la tête fémorale. Le fût est mobile en translation selon son axe longitudinal et peut être enfoncé dans la tête pour appliquer une force par effet de coin sur la paroi latérale du perçage alors qu'elle est maintenue par un contre-appui, jusqu'à ce que la tête casse. La force de rupture est enregistrée et fournit ainsi des informations statistiques permettant une caractérisation des têtes d'un lot et un contrôle de la qualité finale des têtes.

Malheureusement cet appareil ne peut pas être utilisé pour mettre en oeuvre le « Proof test ». En effet, lors de la mise en oeuvre de ce test, des particules métalliques provenant du frottement du fût sur la paroi intérieure du perçage des têtes se déposent sur cette paroi et rendent ces têtes inutilisables pour l'application médicale recherchée.

Par ailleurs, la partie conique du fût se déforme au cours du test et doit être remplacée régulièrement, typiquement tous les deux ou trois tests. Un autre inconvénient de cet appareil résulte de la nature même du test, en ce que l'effet de coin utilisé ne permet pas d'appliquer une pression uniforme sur tout le pourtour de la paroi intérieure du perçage et conduit inévitablement à des concentrations de contraintes dans certaines régions de la tête en raison d'imperfections de surfaces ou autres. Ces concentrations de contraintes augmentent la dispersion des valeurs de charges de ruptures et péjorent ainsi les résultats statistiques obtenus.

Il résulte de cette situation que les fabricants de têtes fémorales en céramique doivent disposer d'un appareil de test de chaque type susmentionné, ce qui est peu pratique, coûteux et nécessite des manipulations de pièces importantes.

Il existe donc dans ce domaine un besoin non encore satisfait pour un dispositif permettant de mettre en oeuvre à la fois le « Proof test » et le test de rupture.

La présente invention a donc pour but principal de remédier aux inconvénients de l'art antérieur susmentionné, en fournissant un dispositif de test ayant une construction simple et économique et permettant la mise en ouvre des deux tests susmentionnés.

A cet effet, l'invention a pour objet un dispositif pour tester des têtes de prothèses fémorales présentant un perçage borgne destiné à recevoir l'extrémité d'une tige de prothèse fémorale, ledit dispositif comportant un bâti pourvu d'un fût faisant saillie à partir du bâti, ledit fût présentant sensiblement une forme complémentaire audit perçage et étant associé à des moyens permettant d'appliquer une pression sur la paroi intérieure du perçage lorsqu'une tête fémorale est emmanchée sur ledit fût, le dispositif étant caractérisé en ce que le fût comprend une chemise étanche ayant au moins une portion de paroi latérale déformable, et une douille emboîtée sur ladite chemise et pourvue de doigts élastiques qui s'étendent sensiblement en regard de ladite portion de paroi déformable de la chemise, et en ce que ladite chemise définit une chambre intérieure en communication avec des moyens de fourniture d'un fluide sous pression de sorte que la pression du fluide est transmise à la paroi intérieure du perçage via ladite paroi déformable et lesdits doigts élastiques.

Ainsi, ce dispositif de test permet de réaliser, par simple réglage de la pression hydrostatique dans la chambre étanche de la chemise, tant le « Proof test » qui nécessite la mise en oeuvre de faibles pressions que le test de rupture qui nécessite la mise en oeuvre de pressions nettement plus élevées. A noter qu'aucune adaptation particulière du dispositif n'est à prévoir pour réaliser l'un ou l'autre des tests, ce qui fait de ce dispositif un appareil polyvalent et pratique.

Selon une caractéristique avantageuse de l'invention, le fût comprend en outre un manchon en matière synthétique recouvrant la douille au moins dans la région des doigts.

L'utilisation de ce manchon permet d'isoler avantageusement la douille, réalisée en métal, de la paroi intérieure du perçage afin d'empêcher le dépôt éventuels de particules de métal sur la paroi intérieure du perçage lors de la mise en oeuvre du « Proof test ». L'utilisation d'un tel manchon permet également de contribuer à une répartition régulière de la pression sur la paroi intérieure du perçage.

D'autres caractéristiques et avantages de la présente invention apparaîtront dans la description suivante d'un mode de réalisation préféré, présenté à titre d'exemple non limitatif en référence aux dessins annexés, dans lesquels :
- la figure 1 déjà décrite montre un appareil de test de têtes de prothèses fémorales selon l'art antérieur ;
- la figure 2 montre une vue en coupe d'un dispositif pour tester des têtes de prothèses fémorales selon l'invention, dispositif étant représenté à l'état de repos ;
- la figure 3 montre une vue en demi-coupe du fût du dispositif de test selon l'invention, ledit fût étant représenté alors que la chemise étanche et la douille sont déformées par un fluide sous pression ;
- la figure 4 est une vue éclatée partiellement en coupe du fût du dispositif de test selon l'invention ; et
- la figure 5 est une vue en bout de la douille du fût du dispositif de test selon l'invention.

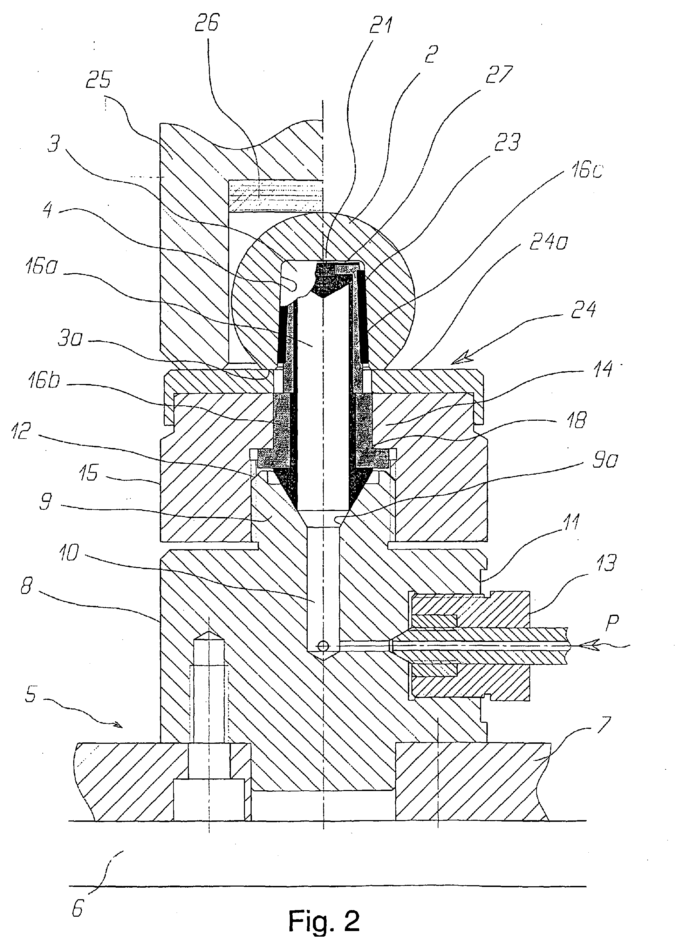

A la figure 2, on voit un dispositif désigné par la référence générale 1 pour tester des têtes 2 de prothèses fémorales selon l'invention. Ces têtes 2 qui sont généralement réalisées en céramique, par exemple en ZrO₂, Al₂O₃, Si₃N₄, présentent la forme générale d'une boule pourvue d'un perçage borgne 3 destiné à recevoir une extrémité d'une tige de prothèse fémorale (non représentée). Le perçage 3 présente le plus souvent une paroi intérieure 4 de forme tronconique.

Le dispositif de test 1 comporte un bâti 5 comprenant une table 6 supportant une plaque de montage 7 qui supporte à son tour un socle 8 terminé par un embout 9 muni d'un filetage extérieur. Le socle 8 comprend un canal 10 qui s'étend à partir de sa paroi latérale 11 pour déboucher sensiblement dans la face d'extrémité 12 de l'embout 9 par un orifice 9a de forme tronconique. L'extrémité du canal 10 qui débouche sur la paroi latérale 11 est reliée au moyen d'un connecteur 13 à une source de pression de fluide P symbolisée par une flèche. Un fût 14 présentant sensiblement une forme complémentaire au perçage 3 est monté fixement sur le socle 8 par l'intermédiaire d'un écrou de fixation 15 pour faire saillie à partir du bâti 5.

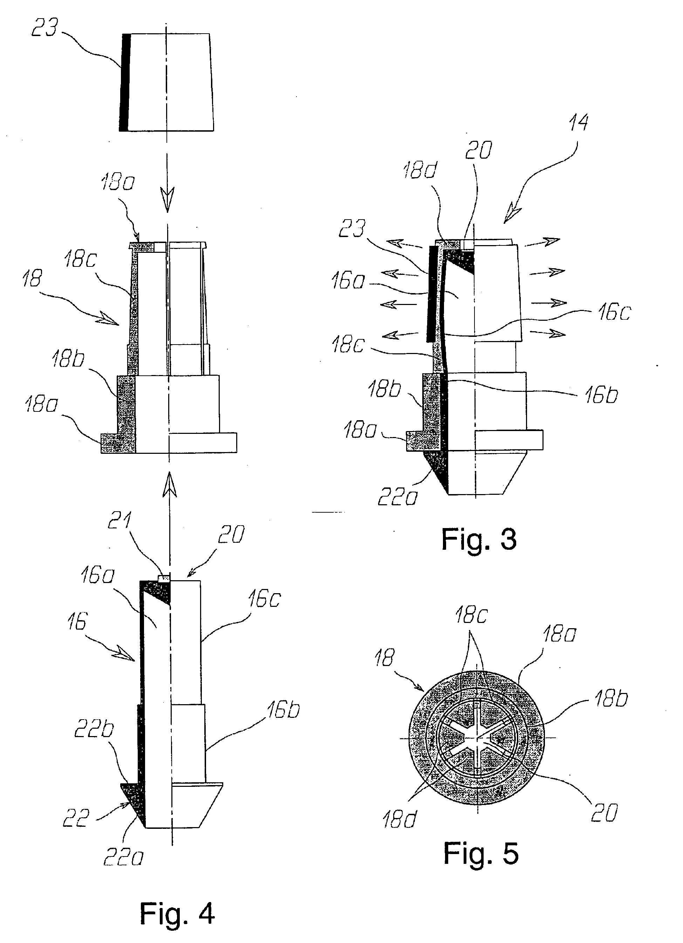

Le fût 14 comprend une chemise étanche 16 (figures 3 et 4) sur laquelle est emboîtée une douille 18. La chemise étanche 16 présente la forme générale d'un cylindre creux fermé à une extrémité par un fond 20 et définit ainsi une chambre 16a. La chemise 16 comprend à son extrémité ouverte un épaulement 22 présentant une première face extérieure 22a tronconique s'étendant depuis son extrémité ouverte, cette première face 22a étant prolongée par une deuxième face extérieure 22b s'étendant perpendiculairement à l'axe longitudinal de la chemise. La première face extérieure 22a est destinée à reposer dans l'orifice 9a de forme complémentaire pour mettre en communication le canal 10 avec l'espace intérieur de la chemise 16. L'appui de ces deux surfaces tronconiques l'une sur l'autre assure ainsi l'étanchéité de la chambre 16a vis-à-vis de l'extérieur. La chemise 16 comprend à partir de l'épaulement 22 une première portion de paroi latérale 16b, typiquement de forme cylindrique, prolongée par une deuxième portion de paroi latérale 16c déformable de préférence élastiquement. La déformabilité de la portion 16c est réalisée par une réduction de l'épaisseur de celle-ci par rapport celle à la première portion de paroi 16b. Compte tenu de la forme cylindrique de la portion 16c , celle-ci prend la forme générale d'un tonneau lorsqu'elle se déforme (figure 3), par exemple suite à la mise sous pression d'un fluide dans la chambre 16a. On notera également que la chemise étanche 16 comprend dans sa partie supérieure, en l'occurrence au niveau du fond 20, des moyens de renfort permettant de limiter la transmission de la pression régnant dans la chambre 16a vers le fond du perçage. Plus précisément, ces moyens de renfort sont intégrés dans le fond 20, qui présente à cet effet une surépaisseur par rapport aux autres portions de paroi de la chemise 16. Dans l'exemple illustré, la surépaisseur a une forme de pointeau dont la pointe est dirigée vers l'intérieur de la chambre 16a. On notera à ce propos que de préférence le fond 20 comprend en outre, sur sa face dirigée vers l'extérieur, un téton de centrage 21 de la douille 18. Le téton 21 peut être formé soit séparément du fond 20, soit venir d'une seule pièce avec ce dernier. Dans le cas où le téton 21 est une pièce séparée du fond 20, il sera de préférence réalisé en matière synthétique.

Pour fixer les idées, la chemise peut être typiquement réalisée en un métal durci tel qu'un acier trempé et l'épaisseur de la portion de paroi déformable peut être de l'ordre de 0,4 mm. Une telle chemise permet de supporter aisément des pressions hydrauliques de l'ordre de 6000 bars.

En se référant également à la figure 4, on voit que la douille 18, emboîtée sur la chemise, comprend un rebord 18a par lequel elle est maintenue fixement sur la chemise 16 au moyens de l'écrou de fixation 15. La douille comprend à partir de ce rebord 18a une première portion cylindrique 18b qui s'étend sensiblement autour de la première portion de paroi latérale 16b de la chemise 16. La portion cylindrique 18b se prolonge ensuite par une pluralité de doigts 18c déformables élastiquement, qui ensemble définissent une forme complémentaire à celle de la chemise 16, en l'occurrence une forme cylindrique. Plus particulièrement, les doigts 18c, au nombre de six dans cet exemple, s'étendent sensiblement en regard de la portion de paroi déformable 16c et sont de préférence en contact intime avec la portion de paroi 16c lorsque la douille 18 est emboîtée sur la chemise 16. Ainsi, toute déformation de la portion de paroi déformable de la chemise est transmise de façon optimale aux doigts 18c de la douille 18 qui, à leur tour, se déforment en s'écartant vers l'extérieur. Dans l'exemple illustré l'extrémité des doigts est recourbée vers l'intérieur de manière à définir une face frontale sensiblement plane 18d (figure 5). On notera que également que cette face frontale 18d comprend un orifice de centrage destiné à coopérer avec le téton de centrage 21 (non représenté à la figure 5) de la chemise 16.

On voit également à la figure 3 que le fût comprend en outre un manchon 23 en matière synthétique recouvrant la douille 18 au moins dans la région des doigts 18c, ce manchon étant déformable élastiquement, et que la face frontale de la douille est également recouverte d'une pastille en matière synthétique 27. A titre d'exemple, le manchon et la pastille peuvent être réalisés en polyéthylène. Ces deux éléments permettent notamment d'éviter un contact direct des parois intérieures du perçage (paroi latérale et fond) avec les éléments métalliques du fût, ce qui permet d'éliminer d'éventuels problèmes de pollution de la tête 2 la rendant ultérieurement inutilisable notamment pour des applications médicales.

Dans une variante de réalisation dans lequel le dispositif est utilisé pour mettre en oeuvre le test de rupture des têtes, le manchon 23 peut être réalisé en métal. Ceci a pour avantage de protéger les doigts 18c en évitant un contact direct avec la paroi latérale du perçage aux pressions élevées mises en oeuvre au cours du test de rupture.

Le dispositif de test 1 comprend en outre une cale 24 en forme de coupelle percée en son centre et ajustée sur l'écrou de fixation 15 de manière à offrir une surface d'appui 24a ayant une hauteur déterminée par rapport au sommet du fût 14. Il est ainsi possible de choisir une cale qui permette, lorsque la tête 2 est emmanchée sur le fût 14, que le rebord périphérique 3a du perçage 3 repose sur la surface d'appui 24a. La coupelle 24 forme ainsi des moyens d'appui de la base de la tête fémorale 2, qui sont réglables en hauteur par rapport au fût 14 pour s'ajuster à différentes profondeurs du perçage 3.

Afin de maintenir la tête 2 en position sur le dispositif 1 lors des tests, le dispositif comprend en outre des moyens de contre-appui 25 pour appliquer la tête fémorale 2 contre la surface d'appui 24a. On notera que ces moyens n'appliquent que la pression nécessaire au maintien en position de la tête 2 sur le fût et n'ont par conséquent pas de rôle dans l'application des pressions de test. Là encore, pour éviter un contact direct entre la tête et un contre appui en métal, une plaquette 26 en matière synthétique, par exemple en polyéthylène, est interposée entre la tête 2 et le contre-appui.

Ainsi lorsqu'une tête fémorale 2 est emmanchée sur ledit fût 14 et que la chambre 16a reçoit un fluide sous pression, la pression du fluide est transmise de la paroi déformable 16c vers les doigts élastiques 18c, qui transmettent à leur tour en s'écartant une pression à la paroi intérieure 4 du perçage 3 (figure 3).

Grâce à la construction qui vient d'être décrite et notamment à la structure du fût 14 en deux pièces métalliques, le cas échéant associées à un manchon synthétique, il est possible de faire varier la pression exercée par le fût sur la paroi latérale intérieure du perçage dans une large gamme de pressions, permettant notamment de mettre en oeuvre le « Proof test » et le test de rupture.

On comprendra que diverses modifications et/ou améliorations évidentes pour un homme du métier peuvent être apportées au mode de réalisation décrit dans la présente description sans sortir du cadre de la présente invention définie par les revendications annexées. On pourra notamment prévoir d'utiliser un fût sans manchon en matière synthétique, ou encore de mettre en oeuvre le dispositif de test sans les moyens de contre-appui. Notamment dans le cas où ces moyens de contre-appui sont utilisés, on pourra prévoir d'intégrer ou d'associer à ces derniers différents capteurs de mesure, par exemple un capteur de mesure de déplacement de la tête au cours du test ou un microphone pour mesurer et/ou détecter des niveaux de bruits découlant de l'apparition ou de la propagation de fissures à l'intérieur de la tête au cours du test.

## Revendications

1. Dispositif pour tester des têtes de prothèses fémorales présentant un perçage borgne destiné à recevoir une extrémité d'une tige de prothèse fémorale, ledit dispositif comportant un bâti pourvu d'un fût faisant saillie à partir du bâti, ledit fût présentant sensiblement une forme complémentaire audit perçage et étant associé à des moyens permettant d'appliquer une pression sur la paroi intérieure du perçage lorsqu'une tête fémorale est emmanchée sur ledit fût, le dispositif étant **caractérisé en ce que** le fût comprend une chemise étanche ayant au moins une portion de paroi latérale déformable, et une douille emboîtée sur ladite chemise et pourvue de doigts élastiques qui s'étendent sensiblement en regard de ladite portion de paroi déformable de la chemise, et **en ce que** ladite chemise définit une chambre intérieure en communication avec des moyens de fourniture d'un fluide sous pression de sorte que la pression du fluide est transmise à la paroi intérieure du perçage via ladite paroi déformable et lesdits doigts élastiques.

2. Dispositif de test selon la revendication 1, **caractérisé en ce que** le fût comprend en outre un manchon recouvrant ladite douille au moins dans la région des doigts.

3. Dispositif de test selon la revendication 2, **caractérisé en ce que** ledit manchon est déformable élastiquement.

4. Dispositif de test selon la revendication 3, **caractérisé en ce que** ledit manchon est réalisé en matière synthétique.

5. Dispositif de test selon la revendication 4, **caractérisé en ce que** le manchon est réalisé en polyéthylène.

6. Dispositif de test selon la revendication 3, **caractérisé en ce que** ledit manchon est réalisé en métal.

7. Dispositif de test selon l'une quelconque des revendications précédentes, **caractérisé en ce que** la chemise étanche comprend dans sa partie supérieure des moyens de renfort permettant de limiter la transmission de la pression régnant dans ladite chambre vers le fond du perçage selon l'axe longitudinal de ladite chemise étanche.

8. Dispositif de test selon l'une quelconque des revendications précédentes, **caractérisé en ce que** la chemise est dimensionnée pour résister à des pressions de l'ordre de 6000 bars.

9. Dispositif de test selon la revendication 1, **caractérisé en ce que** la chemise est réalisée en métal durci.

10. Dispositif de test selon l'une quelconque des revendications précédentes, **caractérisé en ce que** le perçage présente une forme tronconique.

11. Dispositif de test selon l'une quelconque des revendications précédentes **caractérisé en ce qu**'il comprend en outre des moyens d'appui de la base de la tête fémorale qui sont réglables en hauteur par rapport au fût pour s'ajuster à des profondeurs de perçage différentes.

12. Dispositif de test selon la revendication 11, **caractérisé en ce qu**'il comprend en outre des moyens de contre-appui pour appliquer la tête fémorale contre lesdits moyens d'appui.

## Claims

1. Device for testing femoral head prostheses having a blind bore intended to receive one end of a femoral head prosthesis rod, said device comprising a frame provided with a shaft projecting from the frame, said shaft having a substantially complementary shape to that of said bore and being associated with means for applying pressure on said inner wall of the bore when a femoral head is fitted onto said shaft, the device being **characterized in that** the shaft comprises a sealed jacket having at least one deformable lateral wall portion, and a socket fitted onto said jacket and provided with elastic fingers, which extend substantially facing said deformable wall portion of the jacket, and **in that** said jacket defines an inner chamber communicating with means for supplying a pressurized fluid such that the fluid pressure is transmitted to the inner wall of the bore via said deformable wall and said elastic fingers.

2. Test device according to claim 1, **characterized in that** the shaft further comprises a sleeve covering said socket at least in the region of the fingers.

3. Test device according to claim 2, **characterized in that** said sleeve is elastically deformable.

4. Test device according to claim 3, **characterized in that** said sleeve is made of synthetic material.

5. Test device according to claim 4, **characterized in that** the sleeve is made of polyethylene.

6. Test device according to claim 3, **characterized in that** said sleeve is made of metal.

7. Test device according to any one of the previous claims, **characterized in that** the sealed jacket comprises in its upper part reinforcing means for limiting transmission of the pressure prevailing in said chamber towards the bottom of the bore along the longitudinal axis of said sealed jacket.

8. Test device according to any one of the previous claims, **characterized in that** the jacket is dimensioned so as to resist pressures of the order of 6000 bars.

9. Test device according to claim 1, **characterized in that** the jacket is made of hardened metal.

10. Test device according to any one of the previous claims, **characterized in that** the bore has a truncated shape.

11. Test device according to any one of the previous claims, **characterized in that** it further comprises means for supporting the base of the femoral head which are adjustable in height in relation to the shaft in order to fit different bore depths.

12. Test device according to claim 11, **characterized in that** it further comprises counter-support means for applying the femoral head against said support means.

## Patentansprüche

1. Vorrichtung zum Testen von Oberschenkelprothesen, umfassend eine Bohrung, vorherbestimmt um ein Endstück eines Oberschenkelprothesenschaftes aufzunehmen, die besagte Vorrichtung umfasst ein Gestell vorgesehen für einen Säulenschaft, welcher Teil des Gestells ist, der besagte Säulenschaft umfasst deutlich eine komplementäre Form der besagten Bohrung und ist mit Mitteln verknüpft, welche es erlauben, einen Druck auf die Innenwand der Bohrung auszuüben, wenn ein Oberschenkelkopf auf den Säulenschaft aufgesetzt wird, die Vorrichtung ist **dadurch gekennzeichnet, dass** der Säulenschaft einen luftdichten Mantel umfasst, von dem mindestens ein Teil der Innenwand lateral verformbar ist, und eine Buchse eingepasst in den besagten Mantel und vorgesehene elastische Zapfen, die sich deutlich bezogen auf den verformbaren Teil der Innenwand des Mantels ausdehnen, und dieser Mantel definiert eine innere Kammer verbunden mit Beschaffungsmitteln eines Fluids, welches so unter Druck steht, dass der Druck des Fluids auf die Innenwand der Bohrung über die besagte verformbare Innenwand und die besagten elastischen Zapfen übertragen wird.

2. Testvorrichtung nach Anspruch 1, **dadurch gekennzeichnet, dass** der Säulenschaft eine zusätzliche Manschette umfasst, welche die besagte Buchse mindestens im Bereich der Zapfen verkleidet.

3. Testvorrichtung nach Anspruch 2, **dadurch gekennzeichnet, dass** die besagte Manschette elastisch deformierbar ist.

4. Testvorrichtung nach Anspruch 3, **dadurch gekennzeichnet, dass** die besagte Manschette aus synthetischem Material hergestellt ist.

5. Testvorrichtung nach Anspruch 4, **dadurch gekennzeichnet, dass** die Manschette aus Polyethylen hergestellt ist.

6. Testvorrichtung nach Anspruch 3, **dadurch gekennzeichnet, dass** die besagte Manschette aus Metall hergestellt ist.

7. Testvorrichtung nach einem der vorherigen Ansprüche, **dadurch gekennzeichnet, dass** der luftdichte Mantel in seinem oberen Abschnitt Verstärkungsmittel umfasst, welche es erlauben, die Übertragung des vorherrschenden Drucks in der besagten Kammer in Richtung Boden der Bohrung entlang der longitudinalen Achse des besagten luftdichten Mantels zu limitieren.

8. Testvorrichtung nach einem der vorherigen Ansprüche, **dadurch gekennzeichnet, dass** der Mantel dimensioniert ist, um Drücken im Bereich von 6000 bar standzuhalten.

9. Testvorrichtung nach Anspruch 1, **dadurch gekennzeichnet, dass** der Mantel aus gehärtetem Metall gefertigt ist.

10. Testvorrichtung nach einem der vorherigen Ansprüche, **dadurch gekennzeichnet, dass** die vorliegende Bohrung eine kegelförmige Form aufweist.

11. Testvorrichtung nach einem der vorherigen Ansprüche, **dadurch gekennzeichnet, dass** die Vorrichtung zusätzlich Stützmittel an der Basis des Oberschenkelkopfes umfasst, welche in der Höhe gegenüber des Säulenschafts regulierbar sind, um sich an verschiedene Bohrungstiefen anzupassen.

12. Testvorrichtung nach Anspruch 11, **dadurch gekennzeichnet, dass** die Vorrichtung zusätzlich Gegenabstützungsmittel umfasst, um den Oberschenkelkopf gegen die besagten Abstützungsmittel zu applizieren.
